# EUROPEAN PATENT APPLICATION

(11) **EP 4 438 715 A1**
(43) Date of publication of application: **02.10.2024**
(21) Application number: 22898627.9
(22) Date of filing: 24.11.2022
(51) Int. Cl.: C12N 1/06, C07K 16/12, C12M 1/33, C12M 1/34, C12N 1/20, C12N 9/52, C12Q 1/04, C12Q 1/37, G01N 33/48, G01N 33/543, G01N 33/569, C12N 15/13

(54) **BACTERIOLYSIS METHOD AND BACTERIA DETECTION METHOD**

(30) Priority: 24.11.2021 JP 2021190593
(71) Applicant: Asahi Kasei Kabushiki Kaisha, Tokyo 1000006 (JP)
(72) Inventor: TAKAHASHI, Katsuyoshi, Tokyo 100-0006 (JP); FUKUSHIMA, Masakazu, Tokyo 100-0006 (JP)
(74) Representative: Forstmeyer, Dietmar
(86) International application number: PCT/JP2022/043413
(87) International publication number: WO 2023/095845

(57) **Abstract**

Provided is a new bacteriolysis method capable of bacteriolysing multiple species of bacteria and making bacteriolysis treatment more efficient and faster. A bacteriolysis method for lysing multispecies bacterial groups in a specimen, wherein bacteriolysis of multispecies bacterial groups is made possible by a step that reacts a specimen with a surfactant and lytic enzyme in a liquid and bacteriolyses the multispecies bacterial groups present in the specimen.

## Description

### TECHNICAL FIELD

The present invention relates to a bacteriolysis method for lysing a group of two or more species of bacteria in a sample, and a bacterium detection method for detecting a bacterium using the bacteriolysis method. The present invention also relates to a bacteriolysis agent and a bacteriolysis kit to be used for the bacteriolysis method, and a bacterium detection kit to be used for the bacterium detection method.

### BACKGROUND ART

Conventionally, various techniques have been used to lyse bacteria in samples in order to detect bacteria based on their intracellular components. For example, Patent Literatures 1 and 2 (JP2017-032579 A and WO2015/093544 A, respectively) describe methods for detecting Staphylococcus aureus based on its ribosome protein L7/L12 using an antigen-antibody reaction, in which methods lysostaphin is used as a lysing agent to lyse bacteria in the sample. Patent Literature 3 (WO2015/093545 A) describes a method for detecting E. coli based on its ribosome protein L7/L12 using an antigen-antibody reaction, in which method lysozyme is used as a lysing agent to lyse bacteria in the sample.

### LIST OF CITATIONS

### Patent Literature

[Patent Literature 1] JP2017-032579 A
[Patent Literature 2] WO2015/093544 A
[Patent Literature 3] WO2015/093545 A

### SUMMARY OF INVENTION

### Problem to Be Solved by the Invention

However, the conventional bacteriolysis methods as described in Patent Literatures 1 to 3 were not capable of lysing multiple species of bacteria simultaneously. Therefore, when multiple species of bacteria are to be detected simultaneously, it is necessary to use a separate lysing agent for each bacterial genus, resulting in a complicated and delayed bacteriolysis process.

### Means to Solve the Problem

As a result of diligent study, the present inventors have found that multiple species of bacteria can be lysed simultaneously by using a lysis agent containing a surfactant and a lysing enzyme, whereby the bacteriolysis process can be made more efficient and quicker.

Specifically, some aspects of the present invention include the following.
[Aspect 1] A method of lysing a group of two or more species of bacteria including at least one or more species of Gram-positive bacteria and/or Gram-negative bacteria in a sample, comprising the step of treating the sample with a solution containing a surfactant and a bacteriolysis enzyme, wherein the surfactant is selected from amine oxides and quaternary ammoniums.
[Aspect 2] The method according to Aspect 1, wherein the group of two or more species of bacteria includes both at least one or more species of Gram-positive bacteria and at least one or more species of Gram-negative bacteria.
[Aspect 3] The method according to Aspect 1 or 2, wherein the surfactant includes one or more amine oxides.
[Aspect 4] The method according to Aspect 3, wherein the amine oxide is N,N-dimethyldodecylamine N-oxide (DDAO).
[Aspect 5] The method according to Aspect 3 or 4, wherein when the sample is treated with the solution, the concentration of the amine oxide in the solution is from 0.01 to 0.2 mass %.
[Aspect 6] The method according to Aspect 1 or 2, wherein the surfactant includes one or more quaternary ammonium salts.
[Aspect 7] The method according to Aspect 6, wherein the quaternary ammonium salt is benzalkonium chloride.
[Aspect 8] The method according to Aspect 6 or 7, wherein when the sample is treated with the solution the concentration of the quaternary ammonium salt in the solution is from 0.005 to 0.08 mass %.
[Aspect 9] The method according to any one of Aspects 1 to 8, wherein the bacteriolysis enzyme is lysostaphin.
[Aspect 10] The method according to Aspect 9, wherein when the sample is treated with the solution the concentration of the lysostaphin in the solution is from 0.01 µg/mL to 5.0 µg/mL.
[Aspect 11] The method according to any one of Aspects 1 to 10, wherein the Gram-positive bacteria at least includes a bacterium belonging to the genus Staphylococcus.
[Aspect 12] The method according to any one of Aspects 1 to 11, wherein the solution containing the surfactant and the bacteriolysis enzyme contains a dye selected from red, blue, and green.
[Aspect 13] The method according to any one of Aspects 1 to 12, which is used for detecting at least one or more species of bacteria in a sample by immunochromatography.
[Aspect 14] A method for detecting a group of two or more species of bacteria in a sample, comprising the steps of:
   lysing a group of two or more species of bacteria in a sample by the method according to any one of Aspects 1 to 13; and
   detecting an antigen derived from bacteria released from the lysed bacteria group using an antibody that causes an antigen-antibody reaction with the antigen.
[Aspect 15] The method according to Aspect 14, wherein the group of two or more species of bacteria in the sample as the detection target includes both at least one or more species of Gram-positive bacteria and at least one or more species of Gram-negative bacteria.
[Aspect 16] The method according to Aspect 14 or 15, wherein the step of detecting is carried out by immunochromatography.
[Aspect 17] The method according to any one of Aspects 14 to 16, wherein the antigen derived from bacteria released from the step of lysing is an L7 antigen.
[Aspect 18] A bacteriolysis kit to be used in the method according to any one of Aspects 1 to 13 for lysing a group of two or more species of bacteria including at least one or more species of Gram-positive bacteria and/or Gram-negative bacteria in a sample, comprising a surfactant and a bacteriolysis enzyme.
[Aspect 19] A bacteriolysis kit for lysing a group of two or more species of bacteria including at least one or more species of Gram-positive bacteria and/or Gram-negative bacteria in a sample, comprising:
   a surfactant and a bacteriolysis enzyme to be used in the method according to any one of Aspects 1 to 13; and
   an immunochromatographic detection device including
      (a) a labeling antibody-attached member, to which is attached a labeling antibody, which forms a first complex with an antigen derived from the target bacteria based on an antigen-antibody reaction, and
      (b) a strip having a detection region, on which is immobilized a capture antibody, which forms a second complex with the first complex based on an antigen-antibody reaction.
[Aspect 20] A method for detecting the presence or absence and/or the amount of a bacterium in a sample, comprising the steps of:
   (I) lysing bacteria in the sample by the method according to any one of Aspects 1 to 13;
   (II) capturing antigens derived from bacteria in the sample and labeling the antigen derived from the bacterium in the sample based on an antigen-antibody reaction between the sample, a capture antibody immobilized on a solid phase carrier, and a labeling antibody with a label for detection; and
   (III) detecting the bacterium in the sample based on the label for detection,
   wherein one of the capture antibody and the labeling antibody includes one or more generic antibodies, which cause an antigen-antibody reaction with antigens derived from bacteria belonging to five or more genera including the target bacterium, and the other of the capture antibody and the labeling antibody includes one or more specific antibodies, which cause an antigen-antibody reaction with bacteria belonging to one or more genera.
[Aspect 21] The method according to Aspect 20, wherein two or more species of bacteria in the sample are detected in a single capture antibody-immobilized site.

### Effect of the Invention

The present invention makes it possible to lyse multiple species of bacteria (a group of two or more species of bacteria) simultaneously by carrying out bacteriolysis using a surfactant and a lysing enzyme, whereby the bacteriolysis process can be made more efficient and quicker. This also allows for more efficient and rapid detection of bacteria of multiple genera using, e.g., intracellular antigens of bacteria.

### BRIEF EXPLANATION OF FIGURES

[Figure 1] Figure 1 is a cross-sectional view of a schematic diagram of a strip-shaped detection mechanism as an example of a detection mechanism of a lateral flow type immunochromatographic detection system.
[Figure 2] Figure 2 shows the results of test line intensity measurements of samples containing Escherichia coli (EC) or Staphylococcus aureus (SA) detected by immunochromatography, with varying the final concentration of N,N-dimethyldodecylamine N-oxide (DDAO) used as a surfactant.
[Figure 3] Figure 3 shows the results of test line intensity measurements of samples containing Escherichia coli (EC) or Staphylococcus aureus (SA) detected by immunochromatography, with varying the final concentration of benzalkonium chloride used as a surfactant.
[Figure 4] Figure 4 shows the results of test line intensity measurements of samples containing Staphylococcus aureus (SA) detected by immunochromatography using DDAO as a surfactant, with varying the final concentration of lysostaphin used as a bacteriolysis enzyme. Figure 4(a) shows all data, and Figure 4(b) shows data in the region of from 0 to 1.0 µg/ml with expanding the horizontal axis (final concentration of lysostaphin).
[Figure 5] Figure 5 shows the results of test line intensity measurements of samples containing Staphylococcus aureus (SA) detected by immunochromatography using benzalkonium chloride as a surfactant, with varying the final concentration of lysostaphin used as a bacteriolysis enzyme. Figure 5(a) shows all data, and Figure 5(b) shows data in the region of from 0 to 1.0 µg/ml with expanding the horizontal axis.

### DESCRIPTION OF EMBODIMENTS

The present invention will be described in detail in accordance with the specific embodiments below. However, the present invention is not bound by the following embodiments, but can be implemented in any form to the extent that it does not depart from the intent of the present invention.

An embodiment of the present invention relates to a bacteriolysis method for lysing a group of two or more species of bacteria. Another embodiment of the present invention relates to a method of detecting a group of two or more species of bacteria in a sample, including a bacteriolysis step of lysing the group of two or more species of bacteria. Still another embodiment relates to a bacteriolysis agent or a bacteriolysis kit for use in the bacteriolysis method, as well as a bacterium detection kit for detecting a group of two or more species of bacteria in a sample.

### [I. Bacteriolysis method]

An embodiment of the present invention relates to a bacteriolysis method for lysing a group of two or more species of bacteria in a sample (hereinafter also referred to as "the bacteriolysis method of the present invention"). The bacteriolysis method of the present invention includes a bacteriolysis step of treating the sample with a solution containing a surfactant and a bacteriolysis enzyme to thereby lyse a group of two or more species of bacteria existing in the sample. The lysed bacteria group by the bacteriolysis method of the present invention may be subjected to an immunological method. The immunological method may be any detection method using an antigen-antibody reaction, such as immunochromatography, western blotting, ELISA, immunoprecipitation, or immunonephelometry, among which immunochromatography is especially preferred from the viewpoint of facilitating the testing of samples. The group of two or more species of bacteria to be lysed by the bacteriolysis method of the present invention must be lysed so that they can be detected by immunological methods at a later step. Therefore, the bacteriolysis method of the present invention can also be referred to as a bacteriolysis method for the detection by immunological methods.

The bacteriolysis step of lysing a group of two or more species of bacteria in a sample may be carried out by any method as long as it involves treating the sample in a solution containing a surfactant and a bacteriolysis enzyme. As an example, treatment in the solution containing the surfactant and the bacteriolysis enzyme is accomplished by adding a predetermined amount of a solution containing the surfactant and a predetermined amount of a solution containing the lysogenic enzyme to a predetermined volume of the sample. This treatment can also be referred to as a reaction. The reaction of the sample in the solution containing the surfactant and the bacteriolysis enzyme can occur as long as the sample, the surfactant, and the bacteriolysis enzyme co-exist, but the solution may optionally be mixed or agitated. The solution containing the surfactant and the solution containing the bacteriolysis enzyme may be provided either as separate solutions or as a single solution. As another example, at least either or both of the surfactant and the bacteriolysis enzyme may be provided as a dried product, which may be reconstituted in any liquid. The liquid may be a liquid sample. When the sample is liquid, it may be used as a liquid sample as such, or when the sample is solid, it may be suspended in liquid such as buffer and used as a liquid sample. As an example, a dried product of at least either or both of the surfactant and the bacteriolysis enzyme may be added directly to the liquid sample to prepare a solution, or such a dried product may be added to a liquid such as water to prepare a solution, which is then reacted with the sample. A solution or a dried product containing the bacteriolysis enzyme and/or the surfactant may be encapsulated. A dried product or a capsule containing the bacteriolysis enzyme and/or the surfactant may be placed in advance in a container for containing the liquid sample, such that a reaction solution is formed when the liquid sample is put into the container. Alternatively, a dried product or a capsule containing the bacteriolysis enzyme and/or the surfactant may be put into a container and mixed with the liquid sample. As still another example, a dried product or a capsule containing the bacteriolysis enzyme and/or the surfactant may be placed in the pad of an immunochromatographic strip such that when the liquid sample is applied to the strip, its reaction with the solution containing the surfactant and the bacteriolysis enzyme may be accomplished. The reaction time of the sample in the solution containing the surfactant and bacteriolysis enzyme is not limited as long as the bacteriolysis occurs, but may be 10 seconds or more, 30 seconds or more, or one minute or more. The upper limit of the reaction time is also not particularly restricted, but from the viewpoint of speeding up the operation, it may be 1 hour or less, 30 seconds or less, 10 seconds or less, or 5 seconds or less. The sample and the solution containing the surfactant and the bacteriolysis enzyme may further be agitated.

The group of two or more species of bacteria being the subject of bacteriolysis in the bacteriolysis method of the present invention may include, although is not limited to, bacteria selected from at least one or more species of Gram-positive bacteria and at least one or more species of Gram-negative bacteria. More preferably, the group of two or more species of bacteria may contain both at least one or more species of Gram-positive bacteria and at least one or more species of Gram-negative bacteria. Still more preferably, the group of two or more species of bacteria may contain a bacterium of the genus Staphylococcus, which is a Gram-positive bacterium, in addition to one or more other bacteria.

Gram-positive bacteria and Gram-negative bacteria are classes of bacteria that are sharply differentiated by Gram staining, and are known to have significant differences in the composition and structure of cell walls. Specifically, while both classes of bacteria contain peptidoglycans in their cell walls, Gram-positive bacteria have cell walls in which peptidoglycans form a thick layer with tycolic acid, lipotycolic acid, and other proteins, while Gram-negative bacteria have cell walls each include a relatively thin, inner layer, in which peptidoglycans are localized, and an outer layer, which consists of an outer leaflet composed of lipopolysaccharides and an inner leaflet composed of phospholipids.

Due to these major differences in cell wall composition and structure, it has conventionally been extremely difficult to lysate Gram-positive bacteria and Gram-negative bacteria at once with the same bacteriolysis agent. In particular, Staphylococcus spp. cannot be lysed by surfactants or enzymes that can lysate other bacteria, which poses a challenge for lysing a group of two or more species of bacteria. In contrast, the bacteriolysis method of the present invention makes it possible to lysate Gram-positive bacteria and Gram-negative bacteria including Staphylococcus spp. at a time, thereby greatly increasing the efficiency and speed of the bacteriolysis process.

Gram-positive bacteria herein may include, although are not limited to, those belonging to the genera Staphylococcus, Bacillus, Streptococcus, Corynebacterium, Listeria, and Clostridium. Preferred among these may include bacteria belonging to the genera Staphylococcus and Bacillus.

Gram-negative bacteria herein may include, although are not limited to, those belonging to the genera Escherichia, Pseudomonas, Salmonella, Helicobacter, Legionella, Campylobacter, Vibrio, and Yersinia. Preferred among these may include bacteria belonging to the genera Escherichia and Pseudomonas.

The samples to be used in the bacteriolysis method of the present invention may preferably be, although are not limited to, food or environmental samples. Food sample as used herein may be any samples obtained from any food or beverage. As an example, food itself may be used as a food sample, or it may be diluted, ground, swabbed, surface-wiped and/or suspended and used as a food sample. Environmental samples as used herein may be any samples obtained from environments. An example is a sample prepared by wiping the surface of an object with a swab and dispersed in a solution.

In the present invention, any surfactant with bacteriolytic activity can be used as a surfactant, but it may be selected so that it does not interfere with the measurement by immunological techniques after bacteriolysis. For example, sodium dodecyl sulfate is a strong surfactant and can lyse almost all bacteria, but it is not suitable as a surfactant for use in the bacteriolysis method of the present invention because it destroys the structure of antibodies used in immunological techniques. Surfactants that do not interfere with immunological methods after bacteriolysis, especially immunochromatographic measurement, may include, although are not limited to, amine oxide, quaternary ammonium salt, aliphatic amine salt, and betaine. Preferred among these include amine oxides and quaternary ammonium salts.

Examples of amine oxides include, although are not limited to, N,N-dimethyl-C₈₋₂₀ alkyl-amine N-oxide (e.g., N,N-dimethyldodecylamine N-oxide and N,N-dimethylcaprylamine N-oxide), pyridine N-oxide, and N-methylmorpholine N-oxide. Preferred among these include N,N-dimethyldodecylamine N-oxide.

Examples of quaternary ammonium salts include, although are not limited to, benzalkonium chloride, tetramethyl ammonium chloride, tetraethyl ammonium chloride, tetrapropyl ammonium chloride, tetrabutyl ammonium chloride, benzyltrimethyl ammonium chloride, benzyltriethyl ammonium chloride, hexadecyl trimethyl ammonium chloride, benzethonium chloride, benzalkonium chloride, and cetylpyridinium chloride. Preferred among these include benzalkonium chloride.

The concentration of the surfactant in the bacteriolysis according to the present invention is not particularly restricted, but as the concentration in the solution when reacted with the sample, it may preferably be 0.005 mass % or more, or 0.01 mass % or more, or 0.02 mass % or more, and also may preferably be 0.2 mass % or less, or 0.15 mass % or less, or 0.1 mass % or less.

When the surfactant is an amine oxide such as N,N-dimethyldodecylamine N-oxide, the concentration of the amine oxide in the bacteriolysis method of the present invention is not limited, but from the viewpoint of achieving bacteriolysis, it may preferably be 0.01 mass % or more, or 0.025 mass % or more, or 0.05 mass % or more as the concentration in the solution when reacted with the sample. On the other hand, from the viewpoint of maintaining the activity of the enzyme used in combination, it may preferably be 0.2 mass % or less, or 0.15 mass % or less, or 0.1 mass % or less as the concentration in the solution when reacted with the sample.

When the surfactant is a quaternary ammonium salt such as benzalkonium chloride, the concentration of the quaternary ammonium salt in the bacteriolysis method of the present invention is not limited, but from the viewpoint of achieving bacteriolysis, it may preferably be 0.005 mass % or more, or 0.01 mass % or more, or 0.02 mass % or more as the concentration in the solution when reacted with the sample. On the other hand, from the viewpoint of inhibiting aggregation of the labeling antibody, it may preferably be 0.08 mass % or less, 0.075 mass % or less, or 0.065 mass % or less as the concentration in the solution when reacted with the sample.

Examples of bacteriolysis enzymes include, although are not limited to, protein hydrolysis enzymes and carbohydrate hydrolysis enzymes. Examples of protein hydrolysis enzymes include lysostaphin, pepsin, glucosidase, galactosidase, and achromopeptidase. Examples of carbohydrate hydrolysis enzymes include lysozyme and β-N-acetyl glucosaminidase. Preferred among these are protein hydrolysis enzymes, especially lysostaphin.

The concentration of the bacteriolysis enzyme in the bacteriolysis method of the present invention is not restricted, and may be selected as appropriate depending on the type of the bacteriolysis enzyme. However, from the viewpoint of achieving the bacteriolysis effect, in the case of a protein hydrolysis enzyme such as lysostaphin, it may preferably be 0.01 µg/mL or more, or 0.025 µg/mL or more, or 0.05 µg/mL or more, as the concentration in the solution when reacted with the sample. In addition, from the viewpoint of avoiding false positives in immunological testing, it may preferably be 5.0 µg/mL or less, or 3.0 µg/mL or less, or 2.5 µg/mL or less, as the concentration in the solution when reacted with the sample.
the bacteriolysis method of the present invention specifies that the sample is reacted in a solution containing at least one surfactant and at least one bacteriolysis enzyme as identified in the present invention. However, the solution may further contain one or more other ingredients as long as the bacteriolysis lysis effect is not significantly impaired. Such other ingredients include nonionic surfactants, buffers, and bacteriolysis promoters. These components may be added to promote lysis or to allow detection after bacteriolysis.

Nonionic surfactants can be added to ensure flow of the development solution when using immunochromatography after bacteriolysis. Nonionic surfactants are not limited, but ester-ether, ester-type, and ether-type surfactants can all be suitably used. More specific examples include polyoxyethylene alkyl ether, polyoxyethylene alkyl phenyl ether, fatty acid sorbitan esters, alkyl polyglucosides, fatty acid diethanolamides, and alkyl monoglyceryl ethers. Preferred among these include Tween^{™} and Triton^{™}. The concentration of the nonionic surfactant is not limited as long as the concentration does not significantly inhibit the bacteriolysis in the bacteriolysis method of the present invention, the development in the immunochromatographic detection after the bacteriolysis method of the present invention, or the antigen-antibody reaction in the immunoassay method. The concentration of the nonionic surfactant in the liquid at the time of reaction with the sample may preferably be 0.03 mass% or more, 0.05 mass% or more, or 0.1 mass% or more. However, from the viewpoint of not significantly inhibiting the reaction by the bacteriolysis enzyme or the antigen-antibody reaction, the concentration may preferably be 10% or less, 5% or less, or 3% or less.

Although not limited, a buffer solution with a pH of 6.0 to 9.5 may be preferred. From the viewpoint of maintaining the activity of the bacteriolysis enzyme in bacteriolysis, maintaining the antigen-antibody reaction activity in detection after bacteriolysis, and avoiding false positive results, the pH of the buffer may preferably be 6.4 or more, or 6.9 or more, and 9.0 or less, or 8.5 or less. Examples of such buffers include, although are not limited to, Tris buffer, MOPSO buffer, HEPES buffer, citrate buffer, phosphate buffer, and acetate buffer. Preferred among these include Tris-HCl buffer and MOPSO buffer.

The bacteriolysis method of the present invention specifies that the sample is reacted in a solution containing at least one surfactant and at least one bacteriolysis enzyme as identified in the present invention. As an example, a solution containing a surfactant and a bacteriolysis enzyme may be obtained by mixing a solution containing the surfactant and a solution containing the bacteriolysis enzyme. The solution containing the surfactant and the bacteriolysis enzyme may further contain a dye for coloring to prevent accidental swallowing. The coloring may be done by adding the dye to the solution containing the surfactant and/or the solution containing the bacteriolysis enzyme in advance, or by adding a solution containing the dye further when mixing the solution containing the surfactant and the solution containing the bacteriolysis enzyme. The solution containing dye may be a buffer solution. The dye can be selected so as not to interfere with the test to be performed after bacteriolysis. Examples of dyes include those selected from red, blue, yellow, and green dyes. As an example, yellow, blue, or green coloration may be preferred if immunochromatography with gold colloids is used in a later step, because gold colloids exhibit a red coloration. Any dye may be used, but water-water-soluble dyes may be preferred, and food dyes may be preferred from the perspective of ensuring safety in the event that they are orally ingested.

### [II. Bacteriolysis agent]

An embodiment of the present invention relates to a bacteriolysis agent for lysing a group of two or more species of bacteria including at least one or more species of Gram-positive bacteria and/or Gram-negative bacteria in a sample (hereinafter also referred to as the bacteriolysis agent of the present invention). A solution containing the bacteriolysis agent of the present invention may also be referred to as a "reaction solution." The bacteriolysis agent of the present invention contains at least a surfactant and a bacteriolysis enzyme. The bacteriolysis agent of the present invention may be provided either in the form of solution or in the form of a dried product. In particular, the bacteriolysis enzyme may be made into the form of a pad or a capsule and provided in a dried product or a solution with a view to maintaining its activity. The surfactant and the bacteriolysis enzyme may be provided either in the same form or in different forms. The bacteriolysis agent may typically be provided as a single formulation containing both the surfactant and the bacteriolysis enzyme. The bacteriolysis kit may encompass a single formulation containing the surfactant and the bacteriolysis enzyme (i.e., a bacteriolysis agent) and separate formulations containing the surfactant and the bacteriolysis enzyme respectively. The bacteriolysis agent of the present invention may further contain one or more other ingredients as long as the intended lysing effect is not significantly impaired, and may contain dyes to prevent accidental swallowing. Examples of such other ingredients include nonionic surfactants, buffers, and bacteriolysis promoters These components may be added to promote lysis or to allow detection after bacteriolysis. Surfactants, bacteriolysis enzymes, other ingredients, and dyes that may be contained in the bacteriolysis agent are those explained herein.

### [III. Bacteria detection method]

An embodiment of the present invention relates to a method for detecting a group of two or more species of bacteria selected from at least one or more species of Gram-positive bacteria and at least one or more species of Gram-negative bacteria in a sample (hereinafter also referred to as "the bacteria detection method of the present invention"). The bacteria detection method of the present invention includes the step of lysing bacteria belonging to a plurality of genera in a sample by carrying out the bacteriolysis method of the present invention, and the step of detecting an antigen derived from bacteria released from the lysed bacteria group using an antibody that causes an antigen-antibody reaction with the antigen. Therefore, the bacteria detection method of the present invention relates to am immunological method using an antibody. The immunological method may be any detection method using an antigen-antibody reaction, such as immunochromatography, immunoprecipitation method, immunonephelometry, enzyme-linked immunosorbent assay (ELISA), radioimmunoassay (RIA), or fluorescence immunoassay (FIA). Preferred among these include immunochromatography from the viewpoint of facilitating the testing of samples. The group of two or more species of bacteria to be detected by the bacteria detection method of the present invention correspond to a part or all of the bacteria to be lysed by the bacteriolysis method of the present invention.

According to the bacteria detection method of the present invention, the antigen derived from bacteria may preferably be an intracellular antigen. Intracellular antigens are substances that are antigens of the intracellular material of the bacteria released by the bacteriolysis method of the present invention. More specifically, from the viewpoint of detecting bacteria, the intracellular antigen may preferably be a ribosome protein, especially a L7/L12 protein. The L7/L12 ribosomal protein is a type of ribosomal protein that is essential for microbial protein synthesis and is commonly possessed by various bacteria. In addition, L7/L12 ribosomal protein has high detection sensitivity due to the presence of multiple molecules in the cell. Antibodies that produce antigen-antibody reactions against bacterial ribosomal proteins L7/L12 as well as production methods thereof are explained in, e.g., WO2000/006603 A, the publication of an earlier international patent application relating to an invention made by the present inventors. An immunochromatography uses a labeling antibody, which has a label for detection, and a capture antibody, which is immobilized on a strip. The labeling and capture antibodies each bind to an intracellular antigen.

### [Antibodies]

The term "antibody" used herein refers to a protein that recognizes and binds to a specific antigen or substance, which may also be referred to as an immunoglobulin (Ig). Common antibodies typically have two light chains (light chains) and two heavy chains (heavy chains) that are interconnected by disulfide bonds. There are two classes of light chains, called λ and κ chains, and five classes of heavy chains, called γ, µ, α, δ, and ε chains. Depending on the class of their heavy chains, antibodies are classified into five isotypes: IgG, IgM, IgA, IgD and IgE, respectively.

Heavy chains each include a heavy chain constant (CH) region and a heavy chain variable (VH) region. Light chains each include a light chain constant (CL) region and a light chain variable (VL) region. The light chain constant (CL) region consists of a single domain. The heavy chain constant (CH) region consists of three domains, namely CH1, CH2, and CH3. The light chain variable (VL) region and the heavy chain variable (VH) region each consist of four highly conserved regions called framework regions (FRs; FR-1, FR-2, FR-3, and FR-4) and three hypervariable regions called complementarity-determining regions (CDRs; CDR-1, CDR-2, and CDR-3). The heavy chain constant (CH) region consists of three CDRs (CDR-H1, CDR-H2, and CDR-H3) and four FRs (FR-H1, FR-H2, FR-H3, and FR-H4), which are arranged from the amino terminus to the carboxy terminus in the order of FR-H1, CDR-H1, FR-H2, CDR-H2, FR-H3, CDR-H3, and FR-H4. The light chain constant (CL) region has three CDRs (CDR-L1, CDR-L2, CDR-L3) and four FRs (FR-L1, FR-L2, FR L3, and FR-L4), which are arranged from the amino terminus to the carboxy terminus in the order of FR-L1, CDR-L1, FR-L2, CDR-L2, FR-L3, CDR-L3, and FR-L4. The variable regions of the heavy and light chains contain binding domains that interact with the antigen.

The antibody of the present invention may be either a polyclonal antibody or a monoclonal antibody, but a monoclonal antibody be preferred. A polyclonal antibody is usually prepared from the serum of an animal immunized with an antigen and is a mixture of various antibody molecules with different structures. A monoclonal antibody, on the other hand, is an antibody composed of a single type of molecules containing a combination of light chain variable (VL) and heavy chain variable (VH) regions having determined amino acid sequences. Monoclonal antibodies can be produced from clones derived from antibody-producing cells, or they can be produced using genetic engineering technique, by obtaining nucleic acid molecules having gene sequences encoding amino acids of antibody proteins. It is also a well-known technique to those skilled in the art to improve the binding and specificity of antibodies using genetic information of their heavy chains and light chains or their variable regions and CDRs.

The antibody of the present invention may be a fragment and/or derivative of an antibody. Fragments of antibodies include F(ab')₂, Fab, Fv, etc. Antibody derivatives include antibodies in which amino acid mutations have been artificially introduced into the constant region(s) of the light and/or heavy chains, antibodies in which the domain configuration of the constant region(s) of the light and/or heavy chains has been modified, antibodies with two or more Fc regions per molecule, glycosylated antibodies, bispecific antibodies, antibody conjugates in which an antibody or antibody fragment is bound to a protein other than the antibody, antibody enzymes, antibody conjugates in which an antibody or antibody fragment is bound to a protein other than an antibody, etc. Antibody conjugates, antibody enzymes, tandem scFv, bispecific tandem scFv, diabody, etc. Furthermore, when the aforementioned antibodies or their fragments or derivatives are derived from non-human animals, chimeric or humanized antibodies in which some or all of the sequences other than the CDRs thereof are replaced with the corresponding sequences of human antibodies are also included in the scope of the antibody of the present invention. When the simple term "antibodies" is used herein, it is intended to also encompass fragments and/or derivatives of antibodies, unless otherwise specified.

When the antibody of the present invention causes antigen-antibody reactions with certain bacteria, it means that they bind specifically to some components of the bacteria as antigens. The components of bacteria that serve as antigens for the antibody of the present invention are not limited. It may be a component contained in the cell walls or cell membranes that are exposed outside the bacterial cells, or it may be a component contained in the cytoplasm, cell organelles, or nucleus and not exposed outside the bacterial cells.

The degree of antigen-antibody reaction between the antibody of the present invention and the bacteria to be detected is not particularly limited, but the antigen-antibody reaction may occur at least to the extent that it can be detected by some known detection method.

The antibody of the present invention may also preferably not cause any cross-reactions with one or more non-bacterial components that may be present in the sample. Examples of such non-bacterial components include, although are not limited to, various bioorganic compounds derived from viruses, plants, and/or animals that are not present in bacteria. Specific examples of such bioorganic compounds include proteins, sugars, glycoproteins, lipids, complex lipids, and nucleic acids. The antibody of the present invention may preferably not cause any cross-reactions with components derived from at least one, or 2 or more, or 3 or more, or 4 or more, or 5 or more, or 6 or more, or 7 or more, or 8 or more, especially 9 or more, most preferably 10 or more, of these non-bacterial components.

The antibody of the present invention is not limited as long as it can cause an antigen-antibody reaction with the bacteria to be detected, but the generic antibody and/or the specific antibody explained below may be preferably used. Especially when bacterial antigens are detected via a sandwich-type mode, the generic antibody and the specific antibody may preferably be used in combination. The generic antibody may preferably cause an antigen-antibody reaction widely with as many genera of bacteria as possible. Specifically, the generic antibody may cause an antigen-antibody reaction with at least four genera of bacteria, preferably with at least five or more genera of bacteria, more preferably with at least six or more genera of bacteria. The genera of specific bacteria with which the generic antibody causes an antigen-antibody reaction are not limited, but the generic antibody may preferably cause an antigen-antibody reaction with at least bacteria belonging to one or more species of genera selected from the genus Escherichia, the genus Staphylococcus, the genus Pseudomonas, the genus Bacillus, the genus Klebsiella, the genus Serratia, the genus Rahnella, the genus Citrobacter, the genus Listeria, the genus Enterobacter, and the genus Salmonella.

On the other hand, the specific antibody may preferably cause antigen-antibody reactions only with bacteria of limited genera. Specifically, the range of bacteria with which the specific antibody causes antigen-antibody reactions may preferably be commensurate with the range of bacteria to be detected. When only a single specific antibody is used, the range of bacteria for which the specific antibody causes antigen-antibody reactions may be commensurate with the range of bacteria to be detected. On the other hand, when two or more specific antibodies are used in combination, the combined range of bacteria with which the specific antibodies cause antigen-antibody reactions in combination may be commensurate with the range of bacteria to be detected. The latter embodiment is particularly advantageous because it makes it possible to adjust the range of bacteria to be detected in various ways by appropriately combining plural specific antibodies that each cause antigen-antibody reactions with different bacteria.

Each specific antibody of the present invention may cause antigen-antibody reactions with at least one genus of bacteria. The specific bacterial genera with which each specific antibody causes antigen-antibody reactions are not limited, but each specific antibody may preferably cause antigen-antibody reactions at least with bacteria of one or more genera selected from the genus Escherichia, the genus Staphylococcus, Pseudomonas genus (Pseudomonas), the genus Bacillus, the genus Klebsiella, the genus Serratia, the genus Rahnella, the genus Citrobacter, the genus Listeria, the genus Enterobacter, and the genus Salmonella.

### [Labeling antibody]

The labeling antibody is an antibody that is labeled for detection and forms a complex with the antigen derived from the target bacteria released from the lysed target bacteria via an antigen-antibody reaction. The complex thus formed may also be referred to as a first complex. The type of detection label used for the labeling antibody is not restricted and may be selected as appropriate in accordance with the detection method. Specific examples include: metal colloids such as gold colloids, platinum colloids, and palladium colloids; non-metal colloids such as selenium colloids, alumina colloids, and silica colloids; insoluble granular materials such as colored resin particles, dye colloids, colored liposomes, etc.; enzymes that catalyze chromogenic reactions such as alkaline phosphatase, peroxidase, luciferase, etc.; fluorescent dyes, radioisotopes; and chemiluminescent labels, bioluminescent labels, electrochemiluminescent labels, etc. The method for attaching the label to the antibody is also not restricted, but specific examples of methods that can be used include physical adsorption using the hydrophobicity of the antibody, chemisorption using a functional group of the antibody, etc.

### [Capture antibody]

The capture antibody is an antibody that forms a complex with the first complex based on an antigen-antibody reaction. The complex thus formed may also be referred to as a second complex. When used in immunochromatography, the capture antibody may more specifically be immobilized on a membrane support for chromatographic development located in the detection region of the immunochromatographic strip. There are no restrictions on the method used to immobilize the antibody on the solid-phase material. Examples of specific methods include immobilization by physisorption using the hydrophobicity of the antibody and immobilization by chemical bonding using the functional groups of the antibody.

The generic antibody may be the labeling antibody, and the specific antibody may be the capture antibody. Alternatively, the specific antibody may be the labeling antibody, and the generic antibody may be the capture antibody. However, from the viewpoint of facilitating the production of the immunochromatographic detection device, it may be preferable that the generic antibody is the labeling antibody and the specific antibody is the capture antibody.

The second complex is held at the position where the capture antibody is immobilized (capture antibody-immobilized site) via an antigen-antibody reaction with the immobilized capture antibody, and where the labeling of the labeling antibody is detected. If the label is detected at the position where the capture antibody is immobilized, then the target bacteria are detected in the sample solution.

### [IV. Kit]

Another embodiment of the present invention relates to a bacteriolysis kit to be used in the bacteriolysis method of the present invention, for lysing a group of two or more species of bacteria selected from at least one or more species of Gram-positive bacteria and at least one or more species of Gram-negative bacteria in a sample (hereinafter also referred to as "the bacteriolysis kit of the present invention"). The bacteriolysis kit may encompass a single formulation containing a surfactant and a bacteriolysis enzyme (i.e., a bacteriolysis agent) and a combination of separate formulations containing a surfactant and a bacteriolysis enzyme, respectively. The bacteriolysis kit is used for the detection of the group of bacteria after bacteriolysis, and may more preferably be related to a bacteriolysis kit for immunochromatographic detection. The group of bacteria to be lysed, surfactants, and lysing enzymes are as described above.

The bacteriolysis kit may be provided by preparing the surfactant and the bacteriolysis enzyme, either in the form of solution or in the form of solid, and packaging them either in a single container or in two or more separate containers.

Still another embodiment of the present invention may relate to a detection kit to be used in the bacteria detection method of the present invention, for detecting a group of two or more species of bacteria selected from at least one or more species of bacterium, such as a plurality of species of bacteria selected from at least one or more species of Gram-positive bacteria and at least one or more species of Gram-negative bacteria (hereinafter also referred to as "the bacterium detection kit of the present invention"). The bacterium detection kit of the present invention may contain a bacteriolysis kit, or may contain a surfactant and a bacteriolysis enzyme each placed in a device or container for use in a bacterium detection kit. The bacterium detection kit is configured to detect a bacteria group based on an immunological method. The immunological method may be any detection method based on an antigen-antibody reaction, such as immunochromatography, immunoprecipitation method, immunonephelometry, enzyme immunoassay (ELISA), radioimmunoassay (RIA), or fluorescence immunoassay (FIA). Preferred among these from the viewpoint of facilitating the testing of samples is immunochromatography. The group of two or more species of bacteria to be detected using the bacterium detection kit of the present invention correspond to a part or all of the bacteria lysed by the bacteriolysis method of the present invention. The surfactant and the bacteriolysis enzyme to be used for the bacterium detection kit of the present invention are as described above.

The bacterium detection kit of the present invention includes the capture antibody and the labeling antibody explained above. The capture antibody is provided in an appropriate form depending on the type of the solid phase carrier (e.g., a container having a porous membrane, a container having a flow channel, a plate for retaining solution, etc.). The labeling antibody is usually provided in the form of an aqueous reagent containing the labeling antibody in an aqueous medium or a dried reagent in which the labeling antibody is dried.

The bacterium detection kit of the present invention includes, in addition to the capture antibody and the detection antibody explained above, one or more reagents and a detection device or components thereof necessary to perform the method of the present invention using these antibodies, and/or instructions describing the procedure for performing the method of the present invention. The type of such reagents and instructions, as well as other components included in the kit of the present invention, may be determined according to the specific immunological assay used to detect bacteria of plural genera.

When the bacterium detection kit of the present invention contains a device for detection or components thereof, the device assembled from the kit is a device equipped with components necessary for performing the method of the present invention using the labeling antibody and/or the capture antibody of the present invention (hereinafter also referred to as "the device of the present invention"). The specific components of the device of the present invention may be determined according to the type of immunological assay as a specific embodiment of the method of the present invention. As explained above, examples of immunological assay methods using two or more antibodies include, although are not limited to, various known immunological assays such as ELISA using antibody-loaded microtiter plates; latex particle agglutination assay using latex particles (e.g. polystyrene latex particles) loaded with antibodies; immunochromatography using antibody-loaded membranes; and sandwich assay using a detection antibody labeled with colored or chromogenic particles, enzymes or fluorophores, etc., and a capture antibody immobilized on a solid phase carrier such as magnetic particles. Devices equipped with components necessary to perform such various immunological assays may be used as the device of the present invention. Two or more species of bacteria in a sample can also be detected with a single capture antibody-immobilized site. In this case, one or more species are immobilized on a single capture antibody-immobilized site. This single capture antibody-immobilized (linkage) site may be used for detecting two or more species of bacteria in a sample simultaneously (detection of the total amount of bacteria).

Specific examples of immunochromatographic detection devices include devices of the lateral-flow type and those of the flow-through type. According to the lateral-flow devices, the analyte to be detected and the antibody to be detected are deployed parallel onto a membrane having a detection area on the surface of which the capture antibody is immobilized, and the target substance captured in the detection area of the membrane is detected. Lateral flow kits can be generally classified into two main types: dipstick type and cassette type. According to the dipstick type, the sample solution is spread by immersing an immersion area (or a sample pad) of the detection device in the sample solution, whereas according to the cassette type, the sample solution is spread by adding the sample to the sample addition member (sample pad) (3) of the detection device. On the other hand, according to the flow-through devices, the analyte to be detected and the detection antibody are passed vertically through a membrane on which the capture antibody is immobilized on the surface, and the target substance captured on the membrane surface is detected. The method of the present invention can be applied to both lateral-flow devices and flow-through devices.

Both lateral-flow type and flow-through type immunochromatographic detection devices are well known, and the details of such devices can be designed by those skilled in the art based on their technical knowledge, other than those described herein. The following is a description of the schematic configuration of the detection mechanism of the lateral flow type immunochromatographic detection device, with reference to the drawings. However, these are only examples of the schematic configuration of the detection procedure, and the configuration of the lateral-flow type immunochromatographic detection system is not limited in any way to the embodiment illustrated in the figure.

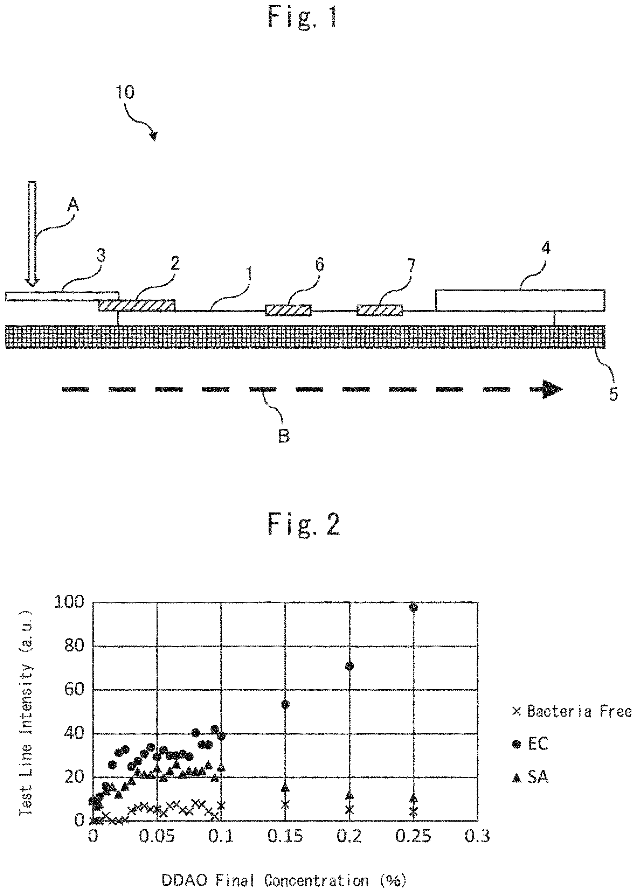

Figure 1 is a cross-sectional view of a schematic configuration of a strip-shaped detection mechanism, which is an example of a detection mechanism of a lateral-flow type immunochromatographic detection system. The immunochromatographic detection device (10) of Figure 1 contains an insoluble membrane carrier (1) for chromatographic development; a strip-shaped labeling antibody-attached member (conjugate pad) (2) (which is impregnated with the labeling antibody) and a sample addition member (sample pad) (3) arranged at one end of the lengthwise direction of the strip (upstream of the sample flow B) on the insoluble membrane carrier (1); and a member for absorption (absorption pad) (4) arranged at the other end (downstream side of the sample flow B) at the other end of the lengthwise direction of the strip (downstream of the sample flow B) on the insoluble membrane carrier (1). The labeling antibody attached to the labeling antibody-attachment member (conjugate pad) (2) may elute into the sample solution. Arranged in the center of the strip lengthwise direction on the insoluble membrane carrier (1) for chromatographic development is a capture antibody-immobilized site (6) on which the capture antibody is immobilized, along with, if necessary, a control reagent-immobilized site (7) on which the control reagent is immobilized. The control reagent is a reagent that does not bind to the analyte but does bind to the detection antibody. A bacteriolysis agent may be placed on this strip to allow elution of the lysing agent into the sample solution.

At the time of use, when a sample A is applied on the sample addition member (sample pad) (3), the sample A passes through the labeling antibody-attached member (conjugate pad) (2) impregnated with the labeling antibody, and flows through the insoluble membrane carrier (1) in the sample flow direction B. During this process, the analyte in the sample (in the case of the present invention, bacteria to be detected) binds to the labeling antibody to form an analyte-labeling antibody complex (first complex). When the sample A passes through the capture antibody-immobilized site (5), the analyte in the sample binds to the capture antibody to form a capture antibody-analyte-labeling antibody complex (second complex). When the sample A passes through the control reagent-immobilized site (7), the labeling antibody that has not bound to the analyte binds to the control reagent (7), whereby it is confirmed that the test has been completed (i.e., that sample A has passed through the capture antibody-immobilized site (6)). The presence or amount of the analyte can be detected by detecting the label of the labeling antibody in the capture antibody-analyte-labeling antibody complex (second complex) that exists in the capture antibody-immobilized site (6) by known means. If necessary, the label of the labeling antibody may be sensitized by known methods to facilitate detection.

The labeling antibody-attached member (conjugate pad) (2), the sample addition member (sample pad) (3), and/or the control reagent-immobilized site (7) may be optionally omitted. When the present mechanism lacks the labeling antibody-attached member (conjugate pad) (2) , the same test as above can be performed by applying the sample A and the labeling antibody to one end on the insoluble membrane carrier (1) for chromatographic development in a pre-mixed state or separately, either simultaneously or sequentially.

Even when the capture antibody and the labeling antibody are interchanged, a detection kit can be constructed to enable the same detection.

Other embodiments of the present invention include Aspects 22 to 34 below.

[22] A method for detecting the presence or absence and/or the amount of a bacterium in a sample, comprising the steps of:
(I) treating the sample with a solution containing a surfactant and a bacteriolysis enzyme;
(II) capturing an antigen derived from bacteria in the sample and labeling the antigen derived from bacteria in the sample based on an antigen-antibody reaction between the sample, a capture antibody immobilized on a solid phase carrier, and a labeling antibody with a label for detection; and
(III) detecting the bacteria in the sample based on the label for detection,
wherein one of the capture antibody and the labeling antibody includes one or more generic antibodies, which cause an antigen-antibody reaction with an antigen derived from bacteria belonging to five or more genera, and the other of the capture antibody and the labeling antibody includes one or more specific antibodies, which cause an antigen-antibody reaction with bacteria belonging to one or more genera.

According to this embodiment, even if a plurality of species of bacteria exist in a sample, they can be lysed at the same time, whereby the bacteriolysis treatment can be carried out more efficiently and rapidly. As a result, the detection process can also be carried out more efficiently and rapidly.

[23] The method according to Aspect 22, wherein the bacteria include at least one or more species of Gram-positive bacteria and at least one or more species of Gram-negative bacteria.

[24] The method according to Aspect 22 or 23, wherein the surfactant is one or more amine oxides.

[25] The method according to Aspect 24, wherein the amine oxide is N,N-dimethyldodecylamine N-oxide (DDAO).

[26] The method according to Aspect 24 or 25, wherein when the sample is treated with the solution, the concentration of the amine oxide in the solution is from 0.01 to 0.2 mass %.

[27] The method according to Aspect 22 or 23, wherein the surfactant is one or more quaternary ammonium salt.

[28] The method according to Aspect 27, wherein the quaternary ammonium salt is benzalkonium chloride.

[29] The method according to Aspect 27 or 28, wherein when the sample is treated with the solution, the concentration of the quaternary ammonium salt in the solution is from 0.005 to 0.08 mass %.

[30] The method according to any one of Aspects 22 to 29, wherein the bacteriolysis enzyme is lysostaphin.

[31] The method according to Aspect 30, wherein when the sample is treated with the solution the concentration of the lysostaphin in the solution is from 0.01 µg/mL to 5.0 µg/mL.

According to the embodiments in Aspects 23 to 31, treatment with a solution of a specific surfactant and a specific enzyme each at a specific concentration enables more reliable bacteriolysis treatment, which not only makes the bacteriolysis treatment more efficient and rapid, but also allows for more reliable detection.

[32] The method according to any one of Aspects 22 to 31, wherein the Gram-positive bacteria include at least a bacterium belonging to the genus Staphylococcus.

[33] The method according to any one of Aspects 22 to 32, wherein the solution containing the surfactant and the bacteriolysis enzyme further contains a dye selected from red, blue, and green.

[34] The method according to any one of Aspects 22 to 33, which is used for detecting at least one or more species of bacteria in a sample by immunochromatography.

### EXAMPLES

The present invention will be described in more detail in the following examples, but these examples are only shown for convenience of explanation, and the present invention is not limited to these examples in any sense.

### Example 1: Production of an immunochromatographic test strip for detecting a group of two or more species of bacteria including at least one or more species of Gram-positive bacteria and/or Gram-negative bacteria

### *Production of Generic Antibody

Pseudomonas aeruginosa was used as the immunogen bacteria. Antibodies against the ribosomal protein L7/L12 of Pseudomonas aeruginosa were produced by referring to the method described in WO2000/06603 A. Specifically, an expression vector incorporating DNA encoding the entire amino acid sequence of ribosomal protein L7/L12 of Pseudomonas aeruginosa was used to transform E. coli, which was cultured using LB medium, etc., and purified as a fusion protein based on a tag sequence derived from the expression vector using an affinity column. The resulting Pseudomonas aeruginosa L7/L12 full-length protein was prepared as an immunogen according to the standard method of hybridoma acquisition, and dissolved in PBS such that the concentration of immunogen was 0.4 mg/mL. Freund's adjuvant was added in the same amount, and used to immunize mice four times so that the immunogen level was 50 µg/dose. After confirming the increase in serum antibody titer by test blood collection, mouse spleen cells were harvested. The excised mouse spleen cells were fused with myeloma cells to obtain a wide variety of hybridomas.

The various hybridomas obtained were cultured in HAT medium, and antibodies in the culture supernatant were screened. Screening was performed by ELISA, and hybridomas that produced antibodies simultaneously reactive with lysates of at least four bacterial species, i.e., Escherichia coli (EC), Staphylococcus aureus (SA), Pseudomonas aeruginosa (PA), and Bacillus subtilis (BS), were selected.

According to the routine method of monoclonal antibody production, the selected hybridomas were cultured in TIL MediaI medium supplemented with 10% fetal bovine serum (FBS), and then injected into the abdominal cavity of mice, from which ascites fluid was collected. The collected ascites fluid was filtered through a filter with a mesh aperture of 0.45 µm after centrifugation to separate suspended solids and red blood cells. The resulting filtrate was passed through a Protein G column to adsorb antibodies, whereby the resulting versatile antibodies were purified from the mouse ascites.

### *Production of Specific Antibody I

Likewise, a wide variety of hybridomas were produced by the same procedure as the procedure for obtaining the Generic Antibody, except that Haemophilus influenzae (HI) was used as the immunogen, and that ribosomal protein L7/L12 of Haemophilus influenzae was used as the immunogen. The resulting hybridomas were screened for those that produced antibodies reactive with lysates of at least two bacteria, i.e., Escherichia coli (EC) and Pseudomonas aeruginosa (PA). The obtained hybridomas were then subjected to the same procedure as that used for obtaining the Generic Antibody, whereby Specificity Antibody I was produced.

### *Production of Specific Antibody II

Likewise, a wide variety of hybridomas were produced by the same procedure as the procedure for obtaining the Generic Antibody, except that Staphylococcus aureus (SA) was used as the immunogen, and that ribosomal protein L7/L12 of Staphylococcus aureus was used as the immunogen. The resulting hybridomas were screened for those that produced antibodies reactive with lysates of at least two bacteria, i.e., Staphylococcus aureus (SA)and Bacillus subtilis (BS). The obtained hybridomas were then subjected to the same procedure as that used for obtaining the Generic Antibody, whereby Specificity Antibody II was produced.

### *Production of Immunochromatographic Test Strips

The following immunochromatographic test strips were prepared using the obtained Generic Antibody as the labeling antibody and the obtained Specific Antibodies I and II as the capture antibodies. The resulting solution was applied to a commercially available nitrocellulose membrane cut to 2.5 cm wide and 15 cm long with 1 µL of solution per cm2 to form one line, and dried to make a membrane carrier for immunochromatographic development.

### *Production of Membrane Carrier for Immunochromatographic Development

The Specific Antibodies I and II were mixed with 10 mM sodium phosphate buffer solution to achieve the antibody concentration of 1.5 mg/mL each, and then combined with a solution of 3% (v/v) trehalose. The resulting solution was applied to a commercially available nitrocellulose membrane cut to 2.5 cm wide and 15 cm long to form one line with a solution concentration of 1 µL/cm², and dried to prepare a membrane carrier for immunochromatographic development.

### *Production of Labeling Antibody using Gold Colloids as Label and Labeling Antibody-Attached Member using Gold Colloids as Label:

A commercially available gold colloid solution (particle size: 60nm) was mixed with 1/10 volume of the Generic Antibody to prepare a solution with an antibody concentration of 0.1mg/mL. The solution was allowed to stand at room temperature for 30 minutes to allow the antibody to bind to the surface of the gold colloidal particles. The solution was then blocked by adding BSA solution such that its final concentration in the gold colloid solution was 0.1%, to thereby prepare a labeling antibody solution using the gold colloid as a label. This antibody solution was soaked into a commercially available glass fiber sheet and dried to make a labeling antibody-attached material using gold colloids as a label.

### *Assembly of Immunochromatographic Test Strips:

In addition to the membrane carrier for immunochromatographic development and using gold colloids as a label labeling antibody-attached member, cotton cloth was prepared as a sample addition member, and a filter paper was prepared as an absorption material. These components were laminated on a commercially available polyethylene substrate, cut into 5 mm wide strips to thereby prepare an immunochromatographic test strip with the same configuration as in Figure 1 (in which the control reagent-immobilized site 7 is omitted).

### Example 2: Evaluation of Bacteriolysis and Detection Performance with Various Compositions of Reaction Solutions (Bacteriolysis Agents)

### *Preparation of Bacterial Solutions for Evaluation:

Escherichia coli (EC) and Pseudomonas aeruginosa (PA) were selected as Gram-negative bacteria, and Staphylococcus aureus (SA) and Bacillus subtilis (BS) as Gram-positive bacteria. These bacteria were each dissolved in saline to prepare solutions with 1e6 cfu/ml each. Normal saline was also used as the bacteria-free solution.

### *Preparation of Various Reaction Solutions:

Various reaction solutions were prepared under the conditions shown in Table 1 using 0.1 M Tris-HCl (pH 7.4) buffer solution with 0.2% Tween 20 for immunochromatographic development as a mother solution. The concentration of each surfactant was 0.1% (the final concentration after mixing with the bacterial solution for evaluation (sample) was 0.05%), and the concentration of each bacteriolysis enzyme was 2.0 µg/ml (the final concentration after mixing with the bacterial solution for evaluation (sample) was 1.0 µg/ml).

**[Table 1]**

| Table 1. Composition conditions of various reaction solutions | | |
|---|---|---|
| Conditions | Surfactant | Bacteriolysis enzyme |
| 2-1 | - | - |
| 2-2 | DDAO | - |
| 2-3 | Benzalkonium chloride | - |
| 2-4 | TritonX-100 | - |
| 2-5 | NP-40 | - |
| 2-6 | CHAPS (cholic acid-type sulfobetaine) | - |
| 2-7 | Zwittergent3-12 (sulfobetaine) | - |
| 2-8 | Softazoline CPB-R (palm oil fatty acid amino acetate betaine) | - |
| 2-9 | Amphitol 24B (Lauryl dimethyl amino acetate betaine) | - |
| 2-10 | Laol XA-60-50 (polyoxyethylene alkyl ether) | - |
| 2-11 | SDS (dodecyl sulfuric acid sodium) | - |
| 2-12 | DDAO | Lysostaphin |
| 2-13 | Benzalkonium chloride | Lysostaphin |
| 2-14 | DDAO | Lysozyme |
| 2-15 | Benzalkonium chloride | Lysozyme |

### *Evaluation of Detection Performance in Immunochromatography:

Bacterial lysis and detection performance by antigen-antibody reaction were evaluated by inserting the immunochromatographic test strip prepared in Example 1 into a 1:1 mixture of the bacterial solution for evaluation and the various reaction solutions described above. The evaluation was made by visually judging the degree of redness of the test line area due to gold colloids. The results are shown in Table 2. Visually positives are indicated as +, visually weakly positives as ±, and visually negatives as -.

**[Table 2]**

| Table 2. Detection results under various reaction solution composition conditions | | | | | |
|---|---|---|---|---|---|
| Conditions | No bacteria | EC | PA | SA | BS |
| 2-1 | - | - | ± | - | ± |
| 2-2 | - | + | + | ± | + |
| 2-3 | - | + | + | ± | + |
| 2-4 | - | - | + | - | + |
| 2-5 | - | - | + | - | ± |
| 2-6 | - | - | + | - | ± |
| 2-7 | - | - | + | - | ± |
| 2-8 | - | - | + | - | ± |
| 2-9 | - | - | + | - | ± |
| 2-10 | - | - | + | - | ± |
| 2-11 | - | - | - | - | - |
| 2-12 | - | + | + | + | + |
| 2-13 | - | + | + | + | + |
| 2-14 | - | + | + | ± | + |
| 2-15 | - | + | + | ± | + |

Under the condition without surfactant (Condition 2-1), only weakly positive signals of PA and BS were observed. This may be due to the fact that Tween 20 added for immunochromatographic development caused a slight lysis of bacteria. On the other hand, under the conditions with various surfactants added (Conditions 2-2 to 2-11), the results were all visually negative for the samples without bacteria, while the results were different for each type of bacteria for the samples with bacteria. In particular, EC and SA were hardly lysed with most surfactants, but with DDAO (Condition 2-2) or benzalkonium chloride (Condition 2-3), bacteriolysis and immunochromatographic detection were possible with positive results for EC and weakly positive results for SA.

Next, an additional lysogenic enzyme (lysostaphin or lysozyme) was added to the reaction solution containing DDAO or benzalkonium chloride to enhance the SA signal (Conditions 2-12 to 2-15). Under conditions with lysostaphin (2-12 and 2-13), a positive signal was observed for the sample with SA, negative for the sample with no bacteria, and positive for the samples with bacteria other than SA. Under conditions with lysozyme (2-14 and 2-15), no changes in results were observed from the conditions without lysozyme (2-2 and 2-3), while the SA signal remained weakly positive.

These results indicate that multiple bacterial groups, including at least one or more Gram-positive and/or Gram-negative bacteria, can be simultaneously lysed and detected by immunochromatography by using DDAO or benzalkonium chloride as a surfactant and lysostaphin as a bacteriolysis enzyme.

### Example 3: Study of DDAO or benzalkonium chloride concentration

### *Preparation of Bacterial Solutions for Evaluation (Samples):

Escherichia coli (EC) was selected as the Gram-negative bacteria and Staphylococcus aureus (SA) as the Gram-positive bacteria, and each was dissolved in saline to prepare a solution with a concentration of 1e5 cfu/ml. Normal saline was also used as the bacteria-free solution.

### *Preparation of Various Reaction Solutions:

0.1M Tris-HCl (pH 7.4) buffer was mixed with 2.0 µg/ml of lysostaphin enzyme (the final concentration after mixing with the bacterial solution for evaluation (sample) was 1.0 µg/ml) and 0.2 mass % Tween20 for immunochromatographic development and used as a mother solution to prepare various reaction solutions with DDAO concentrations of from 0 to 0.5 mass % (the final concentrations after mixing with the bacterial solution for evaluation (sample) were 0 to 0.25 mass %) or benzalkonium chloride concentrations of 0 to 0.5 mass % (the final concentrations after mixing with the bacterial solution for evaluation (sample) were 0 to 0.25 mass %).

### *Evaluation of Detection Performance in Immunochromatography:

Bacterial lysis and detection performance by antigen-antibody reaction were evaluated by inserting the immunochromatographic test strip prepared in Example 1 into a 1:1 mixture of the bacterial solution for evaluation and the various reaction solutions described above. The evaluation was made by photographing the test line area with a camera and quantifying the degree of redness of the test line area due to gold colloids via image processing (from the RGB values, the G value was used as indicating the red absorption. This is indicated as "test line intensity" in the graphs below.). A higher test line intensity indicates a higher degree of redness, and a test line intensity of 10 or higher is considered visually positive.

The relationship between the DDAO concentration and the test line intensity is shown in Figure 2. No failure in immunochromatographic liquid development was observed for the samples with DDAO added at any concentrations. The evaluation results for Escherichia coli (EC) showed that higher final concentrations of DDAO promoted bacteriolysis and resulted in higher test line intensities by antigen-antibody reaction than in the absence of DDAO. The evaluation results for Staphylococcus aureus (SA) showed that higher final concentrations of DDAO promoted bacteriolysis and resulted in higher test line intensities by antigen-antibody reaction than in the absence of DDAO, but showed different behavior depending on the final DDAO concentration. The test line intensities increased with final concentrations of DDAO in the range of 0 to 0.05 mass %, then remained almost constant between 0.05 and 0.1 mass %, and decreased above 0.1 mass% (but still higher test line intensity than without DDAO added). It is deemed that while the synergistic effects of lysostaphin enzyme and DDAO on Staphylococcus aureus lysis were demonstrated when the final DDAO concentration was up to 0.1 mass%, lysostaphin enzyme activity was exhibited to thereby suppress the lysis efficiency and test line intensity when the final DDAO concentration exceeded 0.1 mass%.

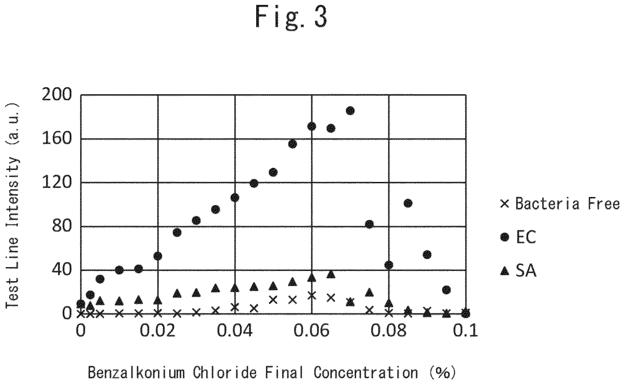

The relationship between the final concentration of benzalkonium chloride and the test line intensity is shown in Figure 3. No failure in immunochromatographic liquid development was observed for the samples with benzalkonium chloride in the range of 0 to 0.05 mass%, but when the final concentration exceeded 0.05 mass%, poor development was observed in part of the liquid flow (gold colloid-labeled antibody was not developed onto the nitrocellulose membrane and remained around the sample addition member), and when the final concentration exceeded 0.075 mass%, the liquid development failure tendency became stronger. The results of the evaluation of Escherichia coli (EC) showed higher bacteriolysis efficiency and test line intensity by antigen-antibody reaction at final concentrations of benzalkonium chloride up to 0.075 mass% compared to no benzalkonium chloride. A final concentration exceeding 0.075 mass% resulted in a decrease in test line intensity due to the aforementioned poor liquid flow development. The evaluation results for Staphylococcus aureus (SA) showed different behavior depending on the benzalkonium chloride final concentration, although the addition of benzalkonium chloride promoted bacteriolysis and resulted in higher test line intensity by antigen-antibody reaction than in the absence of benzalkonium chloride. The test line intensity increased with final concentrations of benzalkonium chloride in the range of 0 to 0.065 mass %, but decreased when the concentration exceeded 0.07 mass %. It is deemed that while the synergistic effects of lysostaphin enzyme and DDAO on Staphylococcus aureus lysis were demonstrated when the final DDAO concentration was up to 0.065 mass%, lysostaphin enzyme activity was exhibited to thereby suppress the lysis efficiency and test line intensity when the final DDAO concentration exceeded 0.07 mass%.

These results indicate that the addition of DDAO as a surfactant, preferably at final concentrations of up to 0.2 mass % and more preferably 0.1 mass %, in combination with lysostaphin enzyme, allows for efficient detection of bacteria of multiple genera (E. coli and Staphylococcus aureus) by bacteriolysis and immunochromatography.

The results also indicate that the addition of benzalkonium chloride as a surfactant, preferably at final concentrations of up to 0.08 mass% and more preferably 0.065 mass%, in combination with lysostaphin enzyme, allows for efficient detection of bacteria of multiple genera (E. coli and Staphylococcus aureus) by bacteriolysis and immunochromatography.

### Example 4: Examination of Lysostaphin Concentration

### *Preparation of Bacterial Solution for Evaluation (Sample):

To see the effect of lysostaphin, Staphylococcus aureus (SA) was dissolved in saline to prepare a solution with a concentration of 1e6 cfu/ml. Normal saline was also used as the bacteria-free solution.

### *Preparation of Various Reaction Solutions:

0.1M Tris-HCl (pH 7.4) buffer was mixed with DDAO at a concentration 0.15 mass % (the final concentration after mixing with the bacterial solution for evaluation (sample) was 0.075 mass %) or benzalkonium chloride at a concentration of 0.05 mass % (the final concentration after mixing with the bacterial solution for evaluation (sample) was 0.025 mass %) as well as 0.2 mass % of Tween20 for immunochromatographic development and used as a mother solution to prepare various reaction solutions with lysostaphin enzyme concentrations of 0 to 50 µg/ml (the final concentration after mixing with the bacterial solution for evaluation (sample) were 0 to 25 µg/ml).

### *Evaluation of Detection Performance in Immunochromatography:

Bacterial lysis and detection performance by antigen-antibody reaction (test line intensity) were evaluated by inserting the immunochromatographic test strip prepared in Example 1 into a 1:1 mixture of the bacterial solution for evaluation and the various reaction solutions described above.

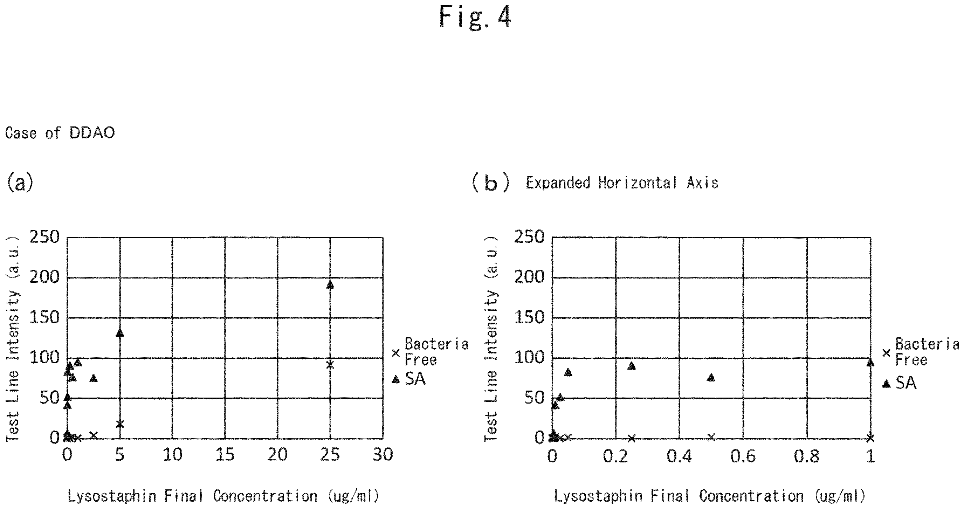

The relationship between the final concentration of lysostaphin and the test line intensity when DDAO is used as a surfactant is shown in Figure 4. Figure 4(a) shows all data, and Figure 4(b) shows data in the region of from 0 to 1.0 µg/ml with expanding the horizontal axis (final concentration of lysostaphin). Compared to the absence of lysostaphin, the addition of lysostaphin tended to promote bacteriolysis of Staphylococcus aureus (SA). When the lysostaphin final concentration exceeded 0.01 µg/ml, sufficient bacteriolysis performance (catalytic action of the enzyme) was observed, resulting in high test line intensity by antigen-antibody reaction. When the lysostaphin final concentration exceeded 5.0 µg/ml, test lines were confirmed (test line intensity ≥ 10) even when no bacteria were present (false positives due to lysostaphin). Therefore, a range of 0.01 to 5.0 µg/ml of lysostaphin final concentration was found to be preferred.

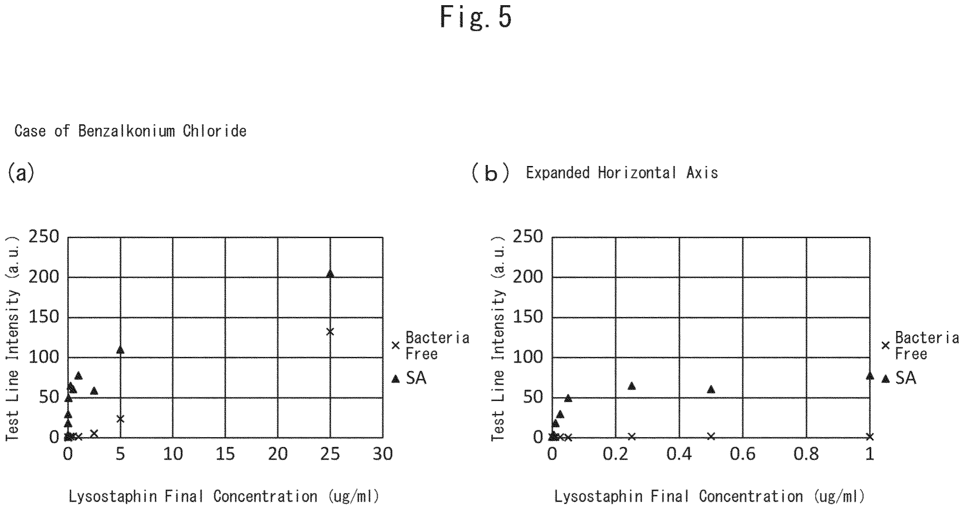

The relationship between the final concentration of lysostaphin and the test line intensity when benzalkonium chloride is used as a surfactant is shown in Figure 5. Figure 5(a) shows all data, and Figure 5(b) shows data in the region of from 0 to 1.0 µg/ml with expanding the horizontal axis. Compared to the absence of lysostaphin, the addition of lysostaphin tended to promote bacteriolysis of Staphylococcus aureus (SA). When the lysostaphin final concentration exceeded 0.01 µg/ml, sufficient bacteriolysis performance (catalytic action of the enzyme) was observed, resulting in high test line intensity by antigen-antibody reaction. When the lysostaphin final concentration exceeded 5.0 µg/ml, test lines were confirmed (test line intensity ≥ 10) even when no bacteria were present (false positives due to lysostaphin). Therefore, a range of 0.01 to 5.0 µg/ml of lysostaphin final concentration was found to be preferred.

These results indicate that the bacteriolysis and immunochromatographic detection of Staphylococcus aureus can be carried out efficiently when lysostaphin is used as a bacteriolysis enzyme at final concentrations of 0.01 µg/ml to 5.0 µg/ml, and in combination with DDAO or benzalkonium chloride as the surfactant.

### Example 5: Examination of Bacteriolysis and Detection Performance near Boundary Conditions

### *Preparation of Various Reaction Solutions:

For confirming the suitable range combination of surfactant DDAO or benzalkonium chloride with lysostaphin enzyme mentioned above, 0.1 M Tris-HCl (pH 7.4) buffer was mixed with 0.2 mass% Tween 20 for immunochromatographic development and used as a mother solution to prepare reaction solutions with the conditions shown in Table 3.

**[Table 3]**

| Table 3. Reaction solution conditions (final concentrations) near boundary conditions | | | | | | | | |
|---|---|---|---|---|---|---|---|---|
| Conditions | 5-1 | 5-2 | 5-3 | 5-4 | 5-5 | 5-6 | 5-7 | 5-8 |
| Lysostaphin (µg/ml) | 0.01 | 0.01 | 1 | 1 | 0.01 | 0.01 | 1 | 1 |
| DDAO (%) | 0.025 | 0.15 | 0.025 | 0.15 | - | - | - | - |
| Benzalkonium chloride (%) | - | - | - | - | 0.005 | 0.05 | 0.005 | 0.05 |

### *Preparation of Bacterial Solutions for Evaluation (Samples):

Escherichia coli (EC) and Pseudomonas aeruginosa (PA) were selected as Gram-negative bacteria, and Staphylococcus aureus (SA) and Bacillus subtilis (BS) as Gram-positive bacteria, and each was dissolved in saline to prepare a solution at a concentration of 1e6 cfu/ml. And as a representative of the bacteria mixture, a solution containing both Escherichia coli (EC) and Staphylococcus aureus (SA) at a concentration of 1e6 cfu/ml was also prepared in saline. Normal saline was also used as the bacteria-free solution.

### *Evaluation of Detection Performance in Immunochromatography:

Bacterial lysis and detection performance by antigen-antibody reaction (test line intensity) were evaluated by inserting the immunochromatographic test strip prepared in Example 1 into a 1:1 mixture of the bacterial solution for evaluation and the various reaction solutions described above. The results are shown in Table 4. In all conditions, the sample with no bacteria was visually negative, while the samples with E. coli (EC), Pseudomonas aeruginosa (PA), Staphylococcus aureus (SA), Bacillus subtilis (BS), and a mixture of E. coli (EC) and Staphylococcus aureus (SA) were visually positive.

**[Table 4]**

| Table 4. Immunochromatography results near boundary conditions | | | | | | | | |
|---|---|---|---|---|---|---|---|---|
| Conditions | 5-1 | 5-2 | 5-3 | 5-4 | 5-5 | 5-6 | 5-7 | 5-8 |
| No bacteria | - | - | - | - | - | - | - | - |
| EC | + | + | + | + | + | + | + | + |
| PA | + | + | + | + | + | + | + | + |
| SA | + | + | + | + | + | + | + | + |
| BS | + | + | + | + | + | + | + | + |
| SA+EC | + | + | + | + | + | + | + | + |

These results indicate that the use of DDAO or benzalkonium chloride as a surfactant in combination with lysostaphin enzyme at suitable concentrations allows for efficient bacteriolysis and immunochromatographic detection of bacteria of multiple genera.

### Example 6: Examination of pH of Reaction Solutions

### *Preparation of Various Reaction Solutions:

0.1M Tris-HCl(or MOPSO) buffer was mixed with 0.15 mass % of DDAO (the final concentration after mixing with the bacterial solution for evaluation (sample) was 0.075 mass %) or 0.05 mass % of benzalkonium chloride (the final concentration after mixing with the bacterial solution for evaluation (sample) was 0.025 mass %) and 5.0 µg/ml of lysostaphin enzyme (the final concentration after mixing with the bacterial solution for evaluation (sample) was 2.5 µg/ml) as well as 0.2 mass % of Tween20 for immunochromatographic development and used as a mother solution to prepare various reaction solutions with different pH values (6.0 to 9.5) via pH adjustment.

### *Preparation of Bacterial Solutions for Evaluation (Samples):

Escherichia coli (EC) and Pseudomonas aeruginosa (PA) were selected as Gram-negative bacteria, and Staphylococcus aureus (SA) and Bacillus subtilis (BS) as Gram-positive bacteria, and each was dissolved in saline to prepare a solution at a concentration of 1e6 cfu/ml. Normal saline was also used as the bacteria-free solution.

### *Evaluation of Detection Performance in Immunochromatography:

Bacterial lysis and detection performance by antigen-antibody reaction (test line intensity) were evaluated by inserting the immunochromatographic test strip prepared in Example 1 into a 1:1 mixture of the bacterial solution for evaluation and the various reaction solutions described above. For the samples at pH 6.0 to 6.2, the test line for Staphylococcus aureus (SA) was negative, and at pH 9.5, the results were weakly positive. At these pH values, the enzymatic activity of lysostaphin appears to be reduced. At other pH values, the samples with no bacteria were visually negative, and the samples with Escherichia coli (EC), Pseudomonas aeruginosa (PA), Staphylococcus aureus (SA), and Bacillus subtilis (BS) were visually positive.

**[Table 5]**

| Table 5. Examination of pH conditions of reaction solutions | | | | | |
|---|---|---|---|---|---|
| pH | No bacteria | EC | PA | SA | BS |
| 6.0 | - | + | + | - | + |
| 6.2 | - | + | + | - | + |
| 6.4 | - | + | + | + | + |
| 6.6 | - | + | + | + | + |
| 6.8 | - | + | + | + | + |
| 7.0 | - | + | + | + | + |
| 7.2 | - | + | + | + | + |
| 7.4 | - | + | + | + | + |
| 7.6 | - | + | + | + | + |
| 7.8 | - | + | + | + | + |
| 8.0 | - | + | + | + | + |
| 8.5 | - | + | + | + | + |
| 9.0 | - | + | + | + | + |
| 9.5 | - | + | + | ± | + |

These results indicate that the use of a pH range of 6.4 to 9.0 for the reaction solution allows efficient lysis and immunochromatographic detection of multiple bacterial groups, including at least one or more Gram-positive and/or Gram-negative bacteria.

### Example 7: Examination of Coloring of Reaction Solutions

### *Selection of Dyes

Coloration of the reaction solution was investigated as a means of preventing accidental swallowing at food sites. Commercially available water-soluble dyes (26 dyes such as blue #1, yellow #4, green #3, etc.) were prepared and their absorption spectra were measured. Four dyes (blue #1, yellow #4, green #3, and a mixture of blue #1 and yellow #4) were selected whose absorption did not interfere with that of the gold colloids (red) used for labeling.

### *Preparation of Colored Reaction Solutions

0.1M Tris-HCl(pH 7.4) buffer was mixed with 0.15 mass % of DDAO (the final concentration after mixing with the bacterial solution for evaluation (sample) was 0.075 mass %) or 0.05 mass % of benzalkonium chloride (the final concentration after mixing with the bacterial solution for evaluation (sample) was 0.025 mass %) and 5.0 µg/ml of lysostaphin enzyme (the final concentration after mixing with the bacterial solution for evaluation (sample) was 2.5 µg/ml) as well as 0.2 mass % Tween20 for immunochromatographic development and used a mother solution, to which the colorants shown in Table 6 were added.

**[Table 6]**

| Table 6: Conditions for colored reaction solution | | | |
|---|---|---|---|
| Conditions | Color | Dye | Added concentration |
| 7-1 | None | None | - |
| 7-2 | Green | Green #3 | 0.001% (final conc. 0.0005%) |
| 7-3 | Blue | Blue #1 | 0.001% (final conc. 0.0005%) |
| 7-4 | Yellow | Yellow #4 | 0.004% (final conc. 0.002%) |
| 7-5 | Green | Blue #1 + | Blue 0.001% (final conc. 0.0005%) |
| | | Yellow #4 | Yellow 0.004% (final conc. 0.002%) |

### *Preparation of Bacterial Solutions for Evaluation (Samples):

Escherichia coli (EC) and Pseudomonas aeruginosa (PA) were selected as Gram-negative bacteria, and Staphylococcus aureus (SA) and Bacillus subtilis (BS) as Gram-positive bacteria, and each was dissolved in saline to prepare a solution at a concentration of 1e6 cfu/ml. Normal saline was also used as the bacteria-free solution.

### *Evaluation of Detection Performance in Immunochromatography:

Bacterial lysis and detection performance by antigen-antibody reaction (test line intensity) were evaluated by inserting the immunochromatographic test strip prepared in Example 1 into a 1:1 mixture of the bacterial solution for evaluation and the various reaction solutions described above. The results are shown in Table 7. As in the case of samples without color, samples with various colors were negative for the absence of bacteria and positive for the presence of bacteria.

**[Table 7]**

| Table 7. Immunochromatographic results | | | | | |
|---|---|---|---|---|---|
| Conditions | No bacteria | EC | PA | SA | BS |
| 7-1 | - | + | + | + | + |
| 7-2 | - | + | + | + | + |
| 7-3 | - | + | + | + | + |
| 7-4 | - | + | + | + | + |
| 7-5 | - | + | + | + | + |

These results indicate that even if the reaction solution is colored as a means of preventing false food and drink, it is possible to efficiently carry out the bacteriolysis and immunochromatographic detection of multiple bacterial groups, including at least one or more Gram-positive and/or Gram-negative bacteria.

### INDUSTRIAL APPLICABILITY

The present invention is widely applicable to the fields involving bacteriolysis and bacterial detection such as food, medicine, etc., and its use is extremely valuable.

### EXPLANATION OF SYMBOLS

- 10: Immunochromatographic detection device
- 1: Membrane carrier for chromatographic development
- 2: Labeling antibody-attached member (conjugate pad)
- 3: Sample addition member (sample pad)
- 4: Absorption member (absorption pad)
- 5: Base material
- 6: Capture antibody-immobilized site
- 7: Control reagent-immobilized site
- A: Sample
- B: Sample flow

## Claims

1. A method of lysing a group of two or more species of bacteria including at least one or more species of Gram-positive bacteria and/or Gram-negative bacteria in a sample, comprising the step of treating the sample with a solution containing a surfactant and a bacteriolysis enzyme, wherein the surfactant is selected from amine oxides and quaternary ammoniums.

2. The method according to claim 1, wherein the group of two or more species of bacteria includes both at least one or more species of Gram-positive bacteria and at least one or more species of Gram-negative bacteria.

3. The method according to claim 1 or 2, wherein the surfactant includes one or more amine oxides.

4. The method according to claim 3, wherein the amine oxide is N,N-dimethyldodecylamine N-oxide (DDAO).

5. The method according to claim 3 or 4, wherein when the sample is treated with the solution, the concentration of the amine oxide in the solution is from 0.01 to 0.2 mass %.

6. The method according to claim 1 or 2, wherein the surfactant includes one or more quaternary ammonium salts.

7. The method according to claim 6, wherein the quaternary ammonium salt is benzalkonium chloride.

8. The method according to claim 6 or 7, wherein when the sample is treated with the solution the concentration of the quaternary ammonium salt in the solution is from 0.005 to 0.08 mass %.

9. The method according to any one of claims 1 to 8, wherein the bacteriolysis enzyme is lysostaphin.

10. The method according to claim 9, wherein when the sample is treated with the solution the concentration of the lysostaphin in the solution is from 0.01 µg/mL to 5.0 µg/mL.

11. The method according to any one of claims 1 to 10, wherein the Gram-positive bacteria at least includes a bacterium belonging to the genus Staphylococcus.

12. The method according to any one of claims 1 to 11, wherein the solution containing the surfactant and the bacteriolysis enzyme contains a dye selected from red, blue, and green.

13. The method according to any one of claims 1 to 12, which is used for detecting at least one or more species of bacteria in a sample by immunochromatography.

14. A method for detecting a group of two or more species of bacteria in a sample, comprising the steps of:
lysing a group of two or more species of bacteria in a sample by the method according to any one of claims 1 to 13; and
detecting an antigen derived from bacteria released from the lysed bacteria group using an antibody that causes an antigen-antibody reaction with the antigen.

15. The method according to claim 14, wherein the group of two or more species of bacteria in the sample as the detection target includes both at least one or more species of Gram-positive bacteria and at least one or more species of Gram-negative bacteria.

16. The method according to claim 14 or 15, wherein the step of detecting is carried out by immunochromatography.

17. The method according to any one of claims 14 to 16, wherein the antigen derived from bacteria released from the step of lysing is an L7 antigen.

18. A bacteriolysis kit to be used in the method according to any one of claims 1 to 13 for lysing a group of two or more species of bacteria including at least one or more species of Gram-positive bacteria and/or Gram-negative bacteria in a sample, comprising a surfactant and a bacteriolysis enzyme.

19. A bacteriolysis kit for lysing a group of two or more species of bacteria including at least one or more species of Gram-positive bacteria and/or Gram-negative bacteria in a sample, comprising:
a surfactant and a bacteriolysis enzyme to be used in the method according to any one of claims 1 to 13; and
an immunochromatographic detection device including
(a) a labeling antibody-attached member, to which is attached a labeling antibody, which forms a first complex with an antigen derived from the target bacteria based on an antigen-antibody reaction, and
(b) a strip having a detection region, on which is immobilized a capture antibody, which forms a second complex with the first complex based on an antigen-antibody reaction.

20. A method for detecting the presence or absence and/or the amount of a bacterium in a sample, comprising the steps of:
(I) lysing bacteria in the sample by the method according to any one of claims 1 to 13;
(II) capturing antigens derived from bacteria in the sample and labeling the antigen derived from the bacterium in the sample based on an antigen-antibody reaction between the sample, a capture antibody immobilized on a solid phase carrier, and a labeling antibody with a label for detection; and
(III) detecting the bacterium in the sample based on the label for detection,
wherein one of the capture antibody and the labeling antibody includes one or more generic antibodies, which cause an antigen-antibody reaction with antigens derived from bacteria belonging to five or more genera including the target bacterium, and the other of the capture antibody and the labeling antibody includes one or more specific antibodies, which cause an antigen-antibody reaction with bacteria belonging to one or more genera.

21. The method according to claim 20, wherein two or more species of bacteria in the sample are detected in a single capture antibody-immobilized site.
